# EUROPEAN PATENT SPECIFICATION

(11) **EP 4 267 889 B1**
(45) Date of publication and mention of the grant of the patent: **01.05.2024**
(21) Application number: 22801225.8
(22) Date of filing: 11.10.2022
(51) Int. Cl.: F24F 3/167, B01L 1/02, B01L 1/04, B25J 21/00, B25J 9/00, E04B 2/74

(54) **MACHINE FOR PHARMACEUTICAL OR BIOTECHNOLOGICAL PROCESSES, ASSEMBLY AND METHOD FOR REALISING THE MACHINE**
MASCHINE FÜR PHARMAZEUTISCHE ODER BIOTECHNOLOGISCHE VERFAHREN, ZUSAMMENBAU UND VERFAHREN ZUR REALISIERUNG DER MASCHINE
MACHINE POUR PROCEDES PHARMACEUTIQUES OU BIOTECHNOLOGIQUES, INSTALLATION ET PROCEDE DE REALISATION DE LA MACHINE

(30) Priority: 13.10.2021 EP 21202343
(43) Date of publication of application: 01.11.2023
(73) Proprietor: Pharma Integration S.R.L., 53100 Siena (IT)
(72) Inventor: BECHINI, Claudio, 53100 Siena (IT)
(74) Representative: Dall'Olio, Christian
(86) International application number: PCT/IB2022/059735
(87) International publication number: WO 2023/062530

(56) References cited:
- EP-A1- 3 335 844
- WO-A1-2021/058616
- US-A- 4 696 902

## Description

### FIELD OF THE INVENTION

The present invention concerns the technical sector relating to the handling of material in chambers having a controlled and/or classified and/or certified atmosphere for realising one or more of the required operations in the life cycle of pharmaceutical or biotechnological articles, such as, for example, handling pharmaceutical or biotechnological material, handling containers and the closing means thereof, verifying or predisposing.

### DESCRIPTION OF THE PRIOR ART

In the technical sector of reference, use is known of process chambers having controlled atmosphere which are located internally of a containment structure such as, for example, an isolator or a RABS (acronym of *Restricted-Access Barrier System).*

The isolators are completely closed and sealed, thus enabling the complete isolation of the chamber. They offer a high level of protection against contamination, especially useful for particularly sensitive, dangerous and/or toxic materials. Further, they enable extensive decontamination or cleanliness processes, for example using hydrogen peroxide or other aggressive decontaminants. Isolators usually comprise a transparent wall for enabling observation of the chamber from the outside environment and the wall, or another, can comprise gloves and/or doors for enabling interventions internally of the chamber.

The isolators are typically made of steel, do not envisage entry of personnel and commonly have a volume as small as possible with respect to the needs of the process, even considering the fact that they are expensive to operate.

Usually, RABS comprise a containing wall which surrounds the apparatus but which remains open towards the white chamber. The containing wall can include gloves and/or doors to allow interaction with the work apparatus. The air treatment system can be integral with the one for the white chamber.

It is specified that, using the common terminology in the reference technical sector, in the present patent application the term "air" is not limited to a particular mixture, for example the mixtures can be equally identified as ambient air or nitrogen. However, in the description of the invention the more generic term "fluid" will be used.

In a larger structure, such as a RABS, there is in general the possibility of accessing the chamber directly by personnel or by other machines.

Typically these processes take place internally of cleanrooms, or clean areas, which are designed, maintained and controlled so as to prevent particle contamination of the articles. By way of example, standard ISO 14644-1, as well as European good manufacturing practice, includes a classification of cleanrooms. At present, in cleanrooms, the control of the atmosphere is done by injecting filtered air, for example using HEPA filters.

Machines are known having a horizontal plane, which use suction of air on both sides of the plane or, if wall-mounted, on one side only. The air is used as an airborne transport for any contaminant substances or particles from the cleaner zones towards the recovery areas. The machines often have shady areas or turbulence areas which prevent formation of a single and substantially unidirectional flow. The horizontal plane also acts as a barrier and deposit for contaminant substances or particles. Likewise the members arranged internally of the chambers modify the flow and intercept it, reducing the effects of the flow at least locally. To obviate these drawbacks, solutions are known for the specific configuration, which, however, do not adapt well in the event of a variation of the system configuration, for example modifications in the station or of the containment structure.

The drawbacks of the machines with the horizontal plane are in part obviated by the machines illustrated in patent application EP 3939896 A1 and in patent EP 3335844 B1 which give examples of a chamber with a controlled atmosphere internally of a containment structure. Both chambers are licked by an air flow, in a top-downwards direction, which intends to limit/prevent the deposit of particles on the exposed surfaces of the process chamber. The air is generally filtered both in inlet and outlet and the flow is usually laminar, or substantially laminar, at least over a portion thereof. The deposit of particles is further limited by the arrangement of the robots on a wall, so as to avoid the use of bases. As in the most widespread use, here and in the rest of this document the term "wall" described an element that extends in a vertical direction.

However, in the machine disclosed in patent application US 4696902 A and also in the solutions discussed in EP 3939896 A1 and in patent EP 3335844 B1 there is a continuous presence of limited deposits of particles or, in any case the cleanliness of the process chamber is not fully satisfactory, especially in the light of the tendency to use substances that are more toxic and/or make the regulations more stringent.

Further, all the discussed solutions require design and realisations that are ad *hoc,* so as to be difficult to design and/or manufacture, as well as to customise in good time or at low cost.

### SUMMARY OF THE INVENTION

The present invention intends to obviate one or more drawbacks of the solutions of the prior art.

A first aim of the present invention is to provide a machine that facilitates maintenance of the chamber in clean conditions required by pharmaceutical or biotechnological processes.

A second aim of the present invention is to guarantee high cleanliness conditions also in the areas which are typically more subject to deposits of contaminant substances or particles.

An aim of some embodiments is to have available a space in proximity of the process stations for housing means for supplying energy or means for supplying materials.

A further aim of some embodiments is to facilitate the integration of the ventilation means as well as the openings for the passage of materials.

A non-secondary aim of the invention is to facilitate the construction of the machine, so that it can be rapidly available and/or quickly customisable, as well as preferably being inexpensive.

These and other aims, which will be obvious to the expert in the sector from a reading of the following text, are attained by means of a machine for pharmaceutical or biotechnological processes, an assembly and a method for realising the machine according to the claims.

In accordance with the teachings of the present document, the machine comprises a structure, ventilation means and movement means.

The structure has an upper part, a lower part and, between them, walls comprising a first wall and a second wall opposite the first wall. The upper part, the lower part and the walls delimit a chamber.

The movement means for moving materials and/or instruments are configured to move the materials and/or the instruments in an operating space internally of the chamber.

The ventilation means comprise at least a fluid injection mouth internally of the chamber, superiorly of the operating space, at least a fluid extraction mouth from the chamber, inferiorly of the operating space, and at least a fluid treatment unit suitable for pharmaceutical or biotechnological processes upstream of the at least an injection mouth. The ventilation means move a fluid from the at least an injection mouth to the at least an extraction mouth.

The structure is configured so that the horizontal section of the chamber is constant, or substantially constant, or diminishes while descending from the at least an injection mouth to the at least an extraction mouth.

Advantageously the first wall comprises a first part, a second part distanced from the first part according to a horizontal direction and an inclined part which extends between the first part and the second part, inferiorly of the second part and superiorly of the first part and which is contiguous with the first part and with the second part so as to confine the chamber.

The operating space extends, at least partly, above the inclined part and/or and/or in a volume arranged superiorly of the first part and in front of the inclined part. At least in part this means that the operating space can also embrace other areas.

The inclined part is inclined with respect to a horizontal plane so that the horizontal section of the chamber crossed by the fluid diminishes when falling towards the first part.

As illustrated in the following, the assembly comprises components that are essential for carrying out the pharmaceutical or biotechnological processes and can be coupled to remaining components so as to form the machine of the invention, as illustrated in the method.

### BRIEF DESCRIPTION OF THE DRAWINGS

Specific embodiments of the invention will be described in the following part of the present description, according to what is set down in the claims and with the aid of the accompanying figures, in which:
- figure 1 is a frontal view of an embodiment of a machine for pharmaceutical or biotechnological processes according to the invention;
- figure 2 is a rear view of a figure 1;
- figures from 3 to 9 illustrate transversal sections of embodiments of a machine for pharmaceutical or biotechnological processes according to the invention which exemplify some of the possible combinations of characteristics according to the present description;
- figure 10 is an axonometric frontal view of an embodiment of an assembly according to the invention;
- figures 11 and figure 12 are axonometric rear views of an embodiment of an assembly according to the invention that is substantially similar to the embodiment of figure 10;
- figure 13 is a frontal view, with a trace line of the cutting plane of the following figure, of another embodiment of a machine for pharmaceutical or biotechnological processes according to the invention which has further chambers;
- figure 14 is a section view of figure 13.

### DESCRIPTION OF THE PREFERRED EMBODIMENTS

With reference to the appended figures, reference numeral 1 denotes a machine for pharmaceutical or biotechnological processes.

An embodiment of the machine (1) comprises a structure (10), movement means for moving materials and/or instruments and ventilation means (2).

The structure (10) has an upper part (11), a lower part (12) and, between them, walls (13, 14, 15, 16). The walls comprise a first wall (13) and a second wall (14) which is opposite the first wall (13).

The upper part (11), the lower part (12) and the walls (13, 14, 15, 16) delimit a chamber (100).

The movement means are configured to move the materials and/or the instruments in an operating space (100a) internally of the chamber (100).

The ventilation means (2) comprise at least a fluid injection mouth (21a, 21b) internally of the chamber (100) superiorly of the operating space (100a), at least a fluid extraction mouth (25a, 25b) of fluid from the chamber (100) inferiorly of the operating space (100a) and at least a fluid treatment unit (221) suitable for pharmaceutical or biotechnological processes upstream of the at least an injection mouth (21a, 21b).

The ventilation means (2) move a fluid from the at least an injection mouth (21a, 21b) to the at least an extraction mouth (25a, 25b).

The structure (10) is configured so that the horizontal section (A) of the chamber (100) is constant, or substantially constant, or diminishes descending from the at least an injection mouth (21a, 21) to the at least an extraction mouth (25a, 25b).

The first wall (13) advantageously comprises a first part (131), a second part (132) distanced from the first part (131) according to a horizontal direction (O) and an inclined part (133) which extends between the first part (131) and the second part (132), inferiorly of the second part (132) and superiorly of the first part (131) and which is contiguous with the first part (131) and with the second part (132) so as to confine the chamber (100).

The operating space (100a) extends, at least partly, above the inclined part (133) and/or and/or in a volume arranged superiorly of the first part (131) and in front of the inclined part (133).

The inclined part (133) is inclined with respect to a horizontal plane (H) so that the horizontal section (A) of the chamber (100) crossed by the fluid diminishes when falling towards the first part (131).

The machine (1) of the invention avoids fluid stagnation areas descending towards the at least an extraction mouth (25a, 25b) and facilitates an acceleration of the fluid so as to prevent deposits of contaminant substances or particles and facilitate airborne transport thereof. In particular the advantages are appreciable at the inclined part (133) and around the movement means which are often the cause of obstacles, shaded areas and/or sources of emission of contaminant substances or particles from the mechanical parts of the movement means. Benefits alike to the ones obtained for the movement means are also attained with respect to any functional units (99) of the process, typically arranged on the inclined part (133) and/or brought from the first part (131), as will be discussed in more detail below.

Typically the materials and/or the instruments are moved to realise one or more pharmaceutical or biotechnological processes.

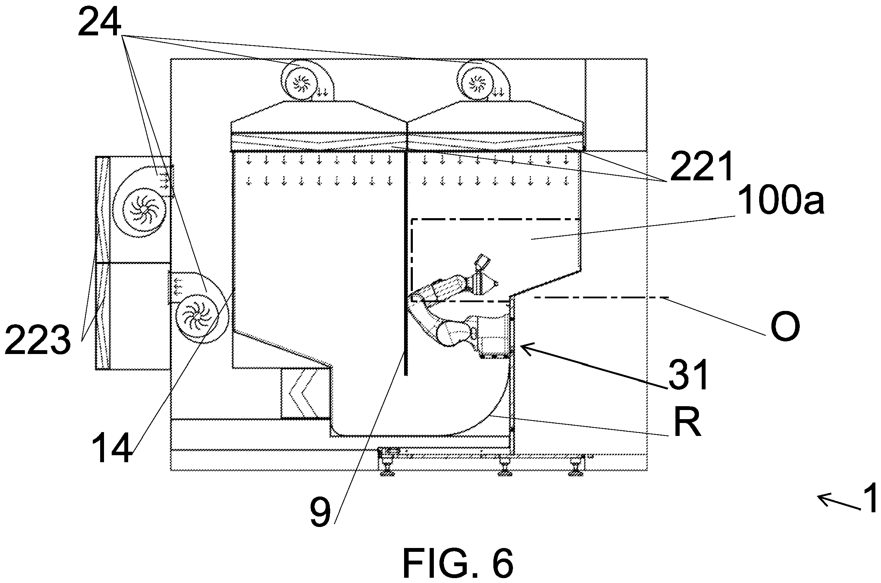

Figures 6, 7 and 8 are examples of some operating spaces (100a) and illustrate how the operating space (100a) does not necessarily correspond to the space that the movement means can reach but to the space where the materials and/or the instruments are in motion.

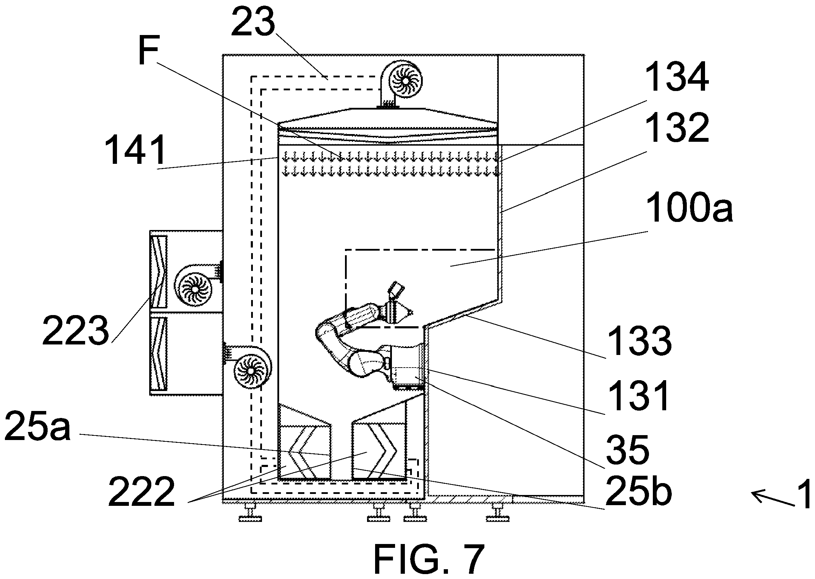

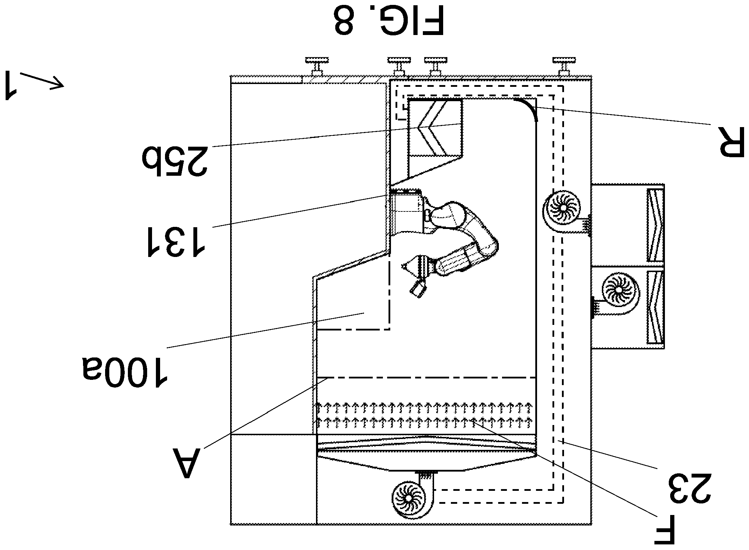

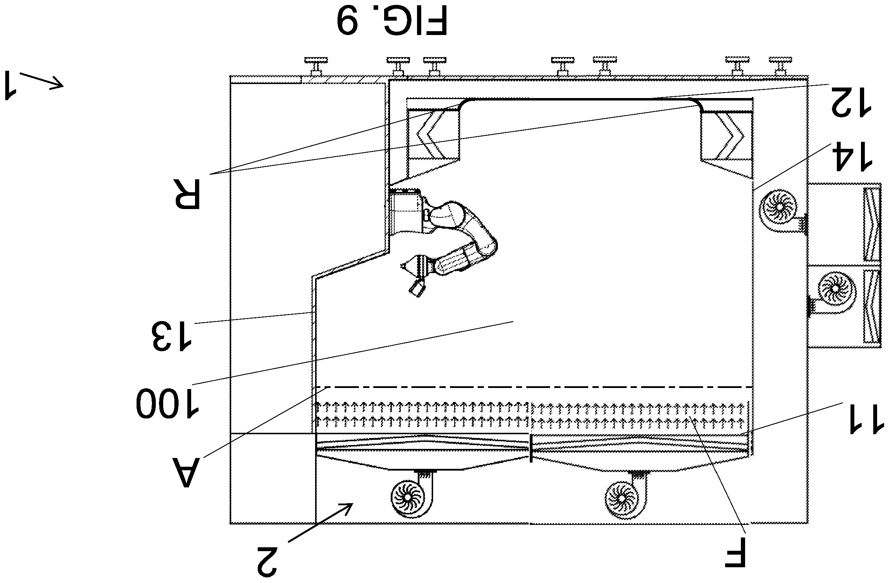

By way of example, the operating space (100a) represented in figure 7 comprises, with respect to the one of figure 8, a volume arranged superiorly of the first part (131) and in front of the inclined part (133); in other words arranged between the inclined part (133) and the second wall (14).

Also in figures 6, 7 and 8 it can be observed how the inclined part (133) is underlying the operating space (100a), i.e. above the inclined part (133).

The operating space (100a) preferably extends, at least partly, above the inclined part (133).

Minimal increasing variations of the horizontal section (A) of the chamber (100) may not influence the velocity of the flow and on the ability thereof to perform airborne transport, in this sense the horizontal section (A) can be substantially constant.

The first wall (13) and the second wall (14) preferably move towards one another, inferiorly of the inclined part (133) and superiorly of the at least an extraction mouth (25a, 25b).

The above-mentioned effects, connected to the increase of the flow velocity, further facilitate airborne transport of the contaminants collected superiorly and the cleanliness of the space involved, where of the movement means or parts of the functional units (99) are often located devices.

Clearly the nearing movement can be determined by the attitude of the first wall (13) and/or of the second wall (14).

The second wall (14) preferably has a part (not illustrated), at the height of the inclined part (133), which is inclined so as to reduce the horizontal section (A).

A further restriction of the horizontal section (A) determined by the second wall (14) internally of the portion corresponding to the inclined part (133) further facilitates the increase of flow velocity with the above-described benefits.

The second part (132) preferably comprises an opening (130) for introducing or removing materials into/from the chamber (100). The machines for pharmaceutical or biotechnological processes are usually provided with similar openings (130) which are often arranged in proximity of the operating space (100a).

The inclined part (133) thus enables directing the flow that laps the opening (130), as well as the contaminants from the materials that can pass through it, towards the at least an extraction mouth (25a, 25b), while at the same time accelerating the contaminants so as to avoid deposits. In this away a high level of cleanliness can be maintained notwithstanding the presence of areas that are openable towards the outside environment or being passed through.

The at least an extraction mouth (25a, 25b) can be made on the first wall (13), as can be seen in figure 8, or on the second wall (14), as shown by way of example in figure 6, or on both, as shown by way of example in figure 7. In combination or alternatively, the at least an extraction mouth (25a, 25b) can be made on the lower part (12). The positioning on the first wall (13) or on the second wall (14) is more convenient especially in the case of filters (222) arranged at the at least an extraction mouth (25a, 25b).

An extraction mouth (25a) is preferably made on the second wall (14).

The extraction from the side opposite to the inclined part (133), inclined, inferiorly thereof, not only facilitates the directionality of the flow, it enables arranging a greater volume beneath the inclined part (133), useful for the installation of apparatus. For example, means for supplying energy (6) and/or means for supplying materials (7) can be installed on the movement means, useful for the pharmaceutical or biotechnological processes that are carried out internally of the chamber (100).

Although the at least an extraction mouth (25a, 25b) can be made on the first wall (13), when realised on the second wall (14) it generally allows affording a greater volume for the filters (222), useful especially on increasing the volume of the chamber (100) and/or for connections to components of the ventilation means (2) arranged on the external side of the second wall (14). This is advantageous in applications where it is difficult to identify further space, beyond the space normally available for the ventilation means (2) above the upper part (11).

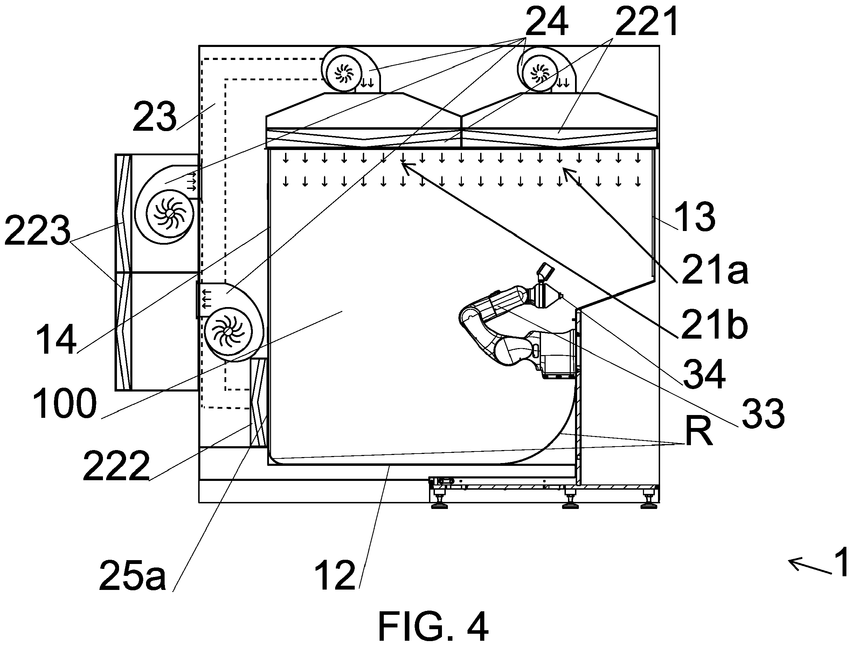

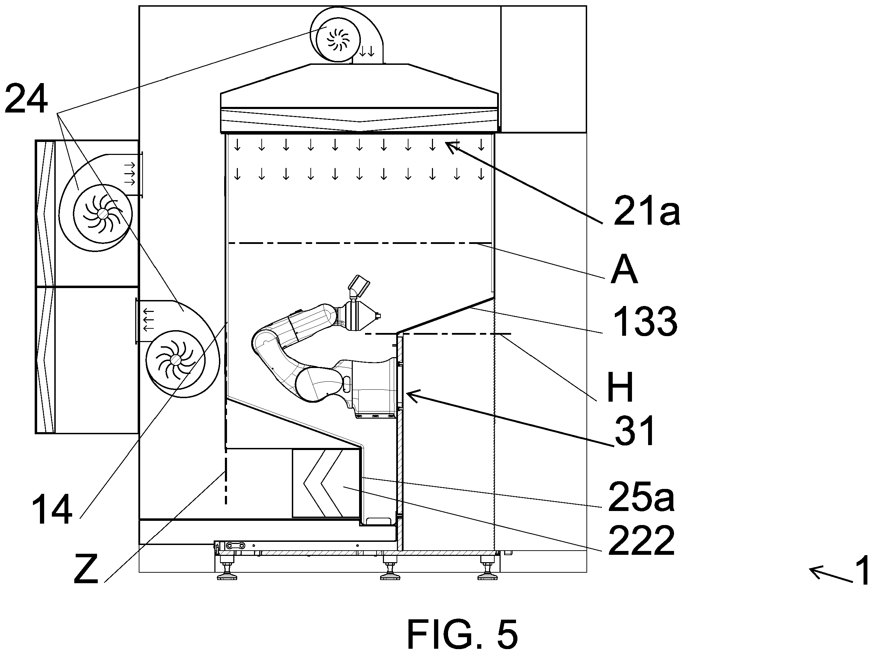

With reference to figures 4 and 5, given same conditions, the chamber (100) of figure 4 requires a filter (222) of greater volume with respect to the filter (222) of the chamber (100) of figure 5.

The first wall (13) is preferably connected to the lower part (12) with a fillet and/or the second wall (14) is connected to the lower part (12) with a fillet, the fillet (R) is conformed so as to direct the flow towards the at least an extraction mouth (25a, 25b), consequently improving airborne transport towards the outside of the chamber (100).

More preferably, in the presence of an extraction mouth (25a) realised on the second wall (14), the first wall (13) is connected to the lower part (12) with a fillet, and, in the presence of an extraction mouth (25b) realised on the first wall (13), the second wall (14) is connected to the lower part (12) with a fillet.

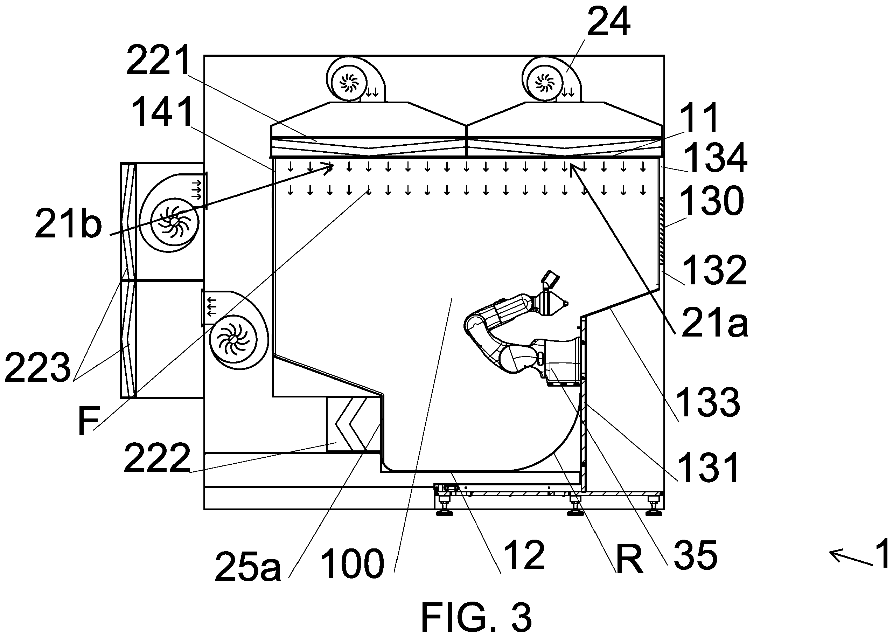

As can be observed in figures 3, 4, 6 and 8, it is preferable for the curvature radius of the fillet (R) to be such that the fillet (R) fronts the at least an extraction mouth (25a, 25b).

The ventilation means (2) preferably comprise a filter (222) suitable for pharmaceutical or biotechnological processes arranged at an extraction mouth (25a, 25b), so as to clean the fluid from the airborne contaminant substances or particles.

The ventilation means (2) preferably comprise a recirculation conduit (23) which connects at least an extraction mouth (25a, 25b) to at least an injection mouth (21a, 21b), as schematically illustrated in figures 4, 7, and 8. More preferably the recirculation conduit (23) passes by the side of the second wall (14), instead of by the side of the first wall (13), as occurs in the known solutions.

The upper part (11) typically houses the at least an injection mouth (21a, 21b) and preferably the ventilation means (2) are configured in such a way that the flow is laminar, or substantially laminar, at least in a first portion (F), and directed perpendicularly to a horizontal plane (H).

Laminar flows are often used in cleanrooms because of the directional aspect thereof and, in the case of the machine (1) of the invention, ensure good airborne transport for a first portion (F) typically free of obstacles, then to be channelled towards extraction. Preferably, especially in the case of a laminar flow, or substantially laminar, at least in a first portion (F), the machine (1) comprises a separator wall (9) of the chamber (100); the separator wall (9) extends from the upper part (11)
towards the lower part (12) to a height of the first part (131).

The separator wall (9) thus realises a configuration alike to a RABS, with a part of the chamber (100) not suffering contaminations of the part of chamber (100) in which the movement means are installed and in which the inclined part (133) is present as well as, possibly, the opening (130).

Preferably, especially in the case of a laminar flow, or substantially laminar, at least in a first portion (F), the at least an injection mouth (21a, 21b) extends, or extends substantially, over all the horizontal section (A) of the chamber (100).

As can be seen especially in figures from 3 to 9, it is preferable that the flow covers the whole horizontal section (A) so as to prevent the presence of areas of contaminant accumulation.

In other words, the at least an injection mouth (21a, 21b) preferably extends, or extends substantially, from the first wall (13) to the second wall (14) and, more preferably, also from another of the walls (15, 16) adjacent to the first wall (13) and the second wall (14).

Especially in the case of a laminar flow, or a substantially laminar flow, at least in a first portion (F), the first wall (13) and the second wall (14) preferably both have a flat vertical part (134, 141) which descends from the upper part (11) and lies on a respective vertical plane (Z). In this way stagnation areas, and possibly the laminar flow does not deviate, modifying the directionality thereof.

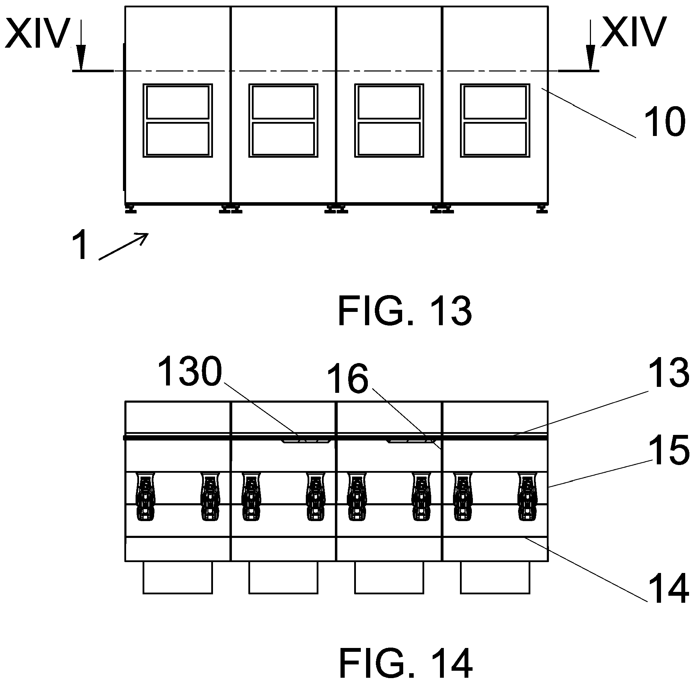

Typically, though not necessarily, the walls (15, 16) adjacent to the first wall (13) and the second wall (14) are flat and vertical, i.e. they lie on vertical planes, as in figure 14; in other embodiments they can contribute to reducing the horizontal section (A).

The movement means can comprise transporters or conveyors, such as, for example, conveyor belts, mechanical arms, robotic arms and/or other known devices.

The movement means preferably comprise an arm (33). The machine (1) of the invention advantageously avoids contaminant accumulation and guarantees airborne transport even in the presence of the arm (33). The arm (33) can be a mechanical arm or a robotic arm.

The movement means typically also comprise a contact part (34), borne by a respective arm (33), which contacts the materials and/or the instruments internally of the operating space (100a).

The movement means can comprise devices borne by the walls (13, 14, 15, 16) and/or by the upper part (11) and/or by the inclined part (133).

Typically, though not necessarily, the movement means comprise a plurality of devices.

The movement means preferably comprise at least a robot (31, 32). More preferably each robot (31, 32) comprises an arm (33) and a contact part (34) and is configured to move the contact part (34) into the operating space (100a).

The movement means, or the at least a robot (31, 32), preferably comprise a part (35) that is coupled to or crosses the first part (131) and/or the inclined part (133) so as to be able to easily receive energy and/or materials.

The machine (1) preferably comprises means for supplying energy (6) to the movement means and/or means for supplying materials (7) to the movement means arranged at the first part (131) or at the inclined part (133) on the side opposite the chamber (100), so that they are in proximity of the operating space (100a).

More preferably, the means for supplying energy (6) and/or the means for supplying materials (7) are located below the inclined part (133), internally of a volume which in the prior art solutions was typically exploited for the passage of air conduits.

The machine (1) preferably comprises at least a functional group (99) which cooperates with the movement means to realise a pharmaceutical or biotechnological process.

The at least a functional group (99) can comprise weighing systems, collecting systems, container-closing systems, ring-sealing stations, liquid or powder dosing systems, parking areas for containers or caps, carousels, centrifuges or other devices commonly used in the pharmaceutical or biotechnological industry.

The inclined part (133) more preferably accommodates or supports at least a functional group (99) and/or the first part (131) supports at least a functional group (99).

In figures 6 and 7 the operating space (100a) enables the movement means to operate above the inclined part (133) and on any functional units (99) born by the first part (131). The definition of the operating space (100a) takes account of the position of the at least a functional group (99) which involves the pharmaceutical or biotechnological process to be carried out.

The ventilation means (2) preferably comprise at least a ventilator (24). Often a ventilator (24) is arranged above the upper part (11) and other ventilators (24) can be introduced to manage overpressure or depression internally of the inside of the chamber (100) as well as the volumes of suction, emission towards the outside and recovery. For example other ventilators (24) are arranged on the side of the second wall (14) in figures from 3 to 9 and enable dynamic management of the recirculation and the pressure internally of the chamber (100).

The at least a fluid treatment unit (221) preferably comprises a filter, more preferably of the HEPA type for pharmaceutical or biotechnological processes.

The ventilation means (2) typically comprise a filter (222) for filtering the air in outlet to the chamber (100), preferably of the HEPA type suitable for pharmaceutical or biotechnological processes.

The ventilation means (2) commonly also comprise a filter (223) for filtering the air in towards the outside or from the outside, preferably of the HEPA type for pharmaceutical or biotechnological processes.

The at least a fluid treatment unit (221) preferably comprises a fluid cooling and/or heating unit.

The means for supplying energy (6) can comprise electric cables, electrical control units, conduits for pressurised fluids or other devices commonly used in the pharmaceutical, biotechnological or robotic industry.

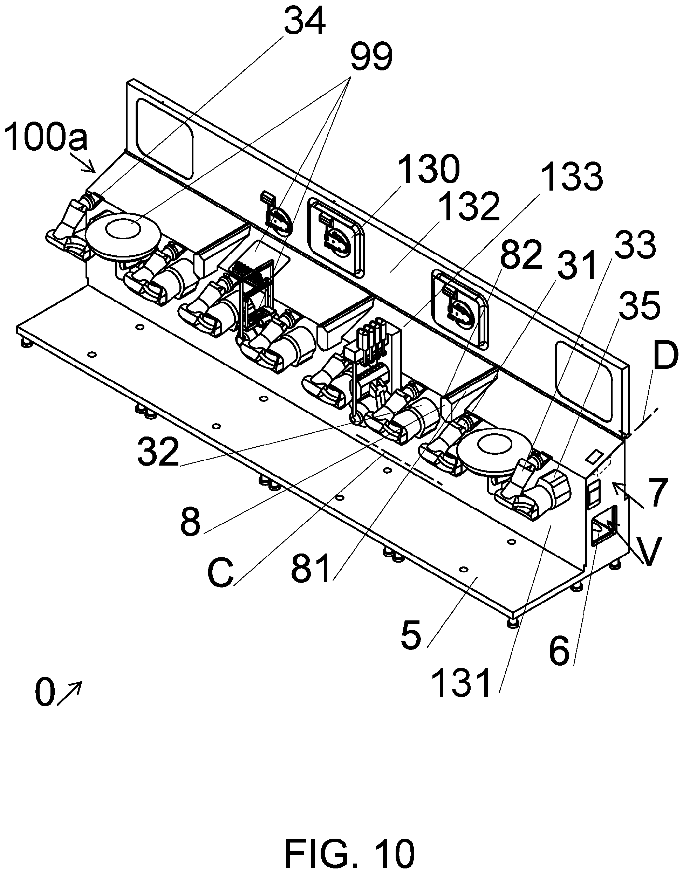

The means for supplying materials (7) can comprise pipes for fluids, such as for example water, air or nitrogen, or mechanical parts for openings and/or drawers and/or compartments, pumps, valves or other devices commonly used in the pharmaceutical or biotechnological industry. By way of example, in figure 10 a compartment open towards the chamber is partially visible, in a dotted line, openable towards the chamber (100).

The materials can comprise containers or parts thereof, pharmaceutical products, biotechnological products, products for tests, or other materials used for pharmaceutical or biotechnological processes.

The instruments can comprise gripping organs, such as, for example pincers, measuring instruments, pick-up devices, filling devices or other instruments used for pharmaceutical or biotechnological processes.

The invention also relates to an assembly (0) for manufacturing the machine (1) according to the present description.

An embodiment of the assembly (0) comprises:
- parts of wall for delimiting a chamber (100) comprising a first part (131) and an inclined part (133);
- at least a further wall (4);
- a base (5) which supports the first part (131) and the at least a further wall (4);
- movement means for moving materials and/or instruments, which are configured to move the materials and/or the instruments in an operating space (100a);
- means for supplying energy (6) to the movement means and/or means for supplying materials (7) to the movement means;
wherein:
- the base (5), the first part (131), the inclined part (133) and the at least a further wall (4) delimit, though not necessarily close, a volume (V) which houses the means for supplying energy (6) and/or the means for supplying materials (7);
- the inclined part (133) is contiguous to the first part (131) and rises from the first part (131) in an oblique direction (D) with respect to a horizontal plane (H).

The assembly (0) according to the invention facilitates the construction of the machine (1) as it comprises components essential for realising pharmaceutical or biotechnological processes in a chamber (100) with the above-delineated advantages.

The assembly (0) can be, for example, pre-validated or pre-qualified and subsequently transported to another place for assembly with the other parts of structure so as to build the machine (1) of the invention.

The assembly (0) can easily be integrated into very different structures to one another, while guaranteeing high levels of cleanliness internally of the chamber (100).

The assembly (0) is further more easily transportable than the whole machine (1) and transport means for delicate or fragile objects can more easily be used.

The parts of wall preferably also comprise a second part (132), more preferably comprising an opening (130), as can be observed for example in figure 10. This can be useful for further limiting the successive operations and maximising the operations before obtaining the prefabricated assembly (0).

The movement means preferably comprise a part (35) that is coupled to or crosses the first part (131) and/or the inclined part (133) so as to be on the opposite side to the volume (V) and to be easily energised or to be able to easily receive materials.

The assembly (0) preferably comprises a separation part (8) and the movement means comprise a first device and a second device.

The separation part (8) is arranged above the inclined part (133) between the first device and the second device and has a first oblique surface (81) and a second oblique surface (82) facing on opposite sides and which broaden towards the inclined part (133) in the conjoining direction (C) of the first device and of the second device.

The separation part (8) can interface with or accommodate a wall (15, 16) so as to delimit therewith the chamber (100) thus facilitating the distancing of the flow from the wall (15, 16) due to the oblique surface, for the same reasons already adopted by the inclined part (133). Likewise, the advantages increase when the wall (15, 16) comprises an opening.

The invention also relates to a method for manufacturing the machine (1) according to the present description.

An embodiment of the method comprises steps of:
- providing an assembly (0) according to the present description, i.e. according to one of the described embodiments;
- providing parts comprising an upper part (11), a lower part (12), walls (13, 14, 15, 16) and a second part (132) of wall configured to form a structure (10) together with the parts of wall, with the upper part (11), the lower part (12) and the walls (13, 14, 15, 16) delimiting the chamber (100);
- providing ventilation means (2) which comprise at least a fluid treatment unit (221) suitable for pharmaceutical or biotechnological processes, at least a fluid injection mouth (21a, 21b) and at least a fluid extraction mouth (25a, 25b);
- connecting the parts to the assembly (0) so as to realise a first wall (13) comprising a first part (131), a second part (132) distanced from the first part (131) according to a horizontal direction (O) and an inclined part (133) which is contiguous with the second part (132) which extends between the first part (131) and the second part (132), inferiorly of the second part (132) and superiorly of the first part (131) and so as to realise a structure (10) having a horizontal section (A) of the chamber (100) which is constant, or substantially constant, or diminishes descending from the installation area of the at least an injection mouth (21a, 21b) to the installation area of the at least an extraction mouth (25a, 25b);
- installing the ventilation means (2) so as to have the at least a fluid injection mouth (21a, 21b) superiorly of the operating space (100a) and the at least a fluid extraction mouth (25a, 25b) from the chamber (100) inferiorly of the operating space (100a) and so as to move a fluid from the at least an injection mouth (21a, 21b) to the at least an extraction mouth (25a, 25b).

The above-described method enables exploiting the advantages of having a prefabricated assembly (0), typically pre-qualified, to form a chamber (100) suitable for the specific requirements of the particular pharmaceutical or biotechnological process. The machine (1) of the invention thus becomes available rapidly and/or can be personalised quickly, as well as having lower costs with respect to those deriving from zero design and manufacturing operations.

Further, the improvements on the assembly (0) can have direct repercussions on all the machines (1) that will be made.

In the step of providing an assembly (0), an assembly is preferably provided that comprises a separation part (8), with the movement means comprising a first device and a second device.

The separation part (8) is arranged above the inclined part (133) between the first device and the second device and has a first oblique surface (81) and a second oblique surface (82) facing on opposite sides and which broaden towards the inclined part (133) in the conjoining direction (C) of the first device and of the second device.

Further, in the step of connecting the parts to the assembly (0), a separation part (8) is connected to a wall (13, 14, 15, 16) in such a way that the wall (13, 14, 15, 16) extends between the first oblique surface (81) and the second oblique surface (82). The above-illustrated advantages are obtained in this way.

The accompanying figures enable clarification with examples and, possible, to detail the above-illustrated teachings.

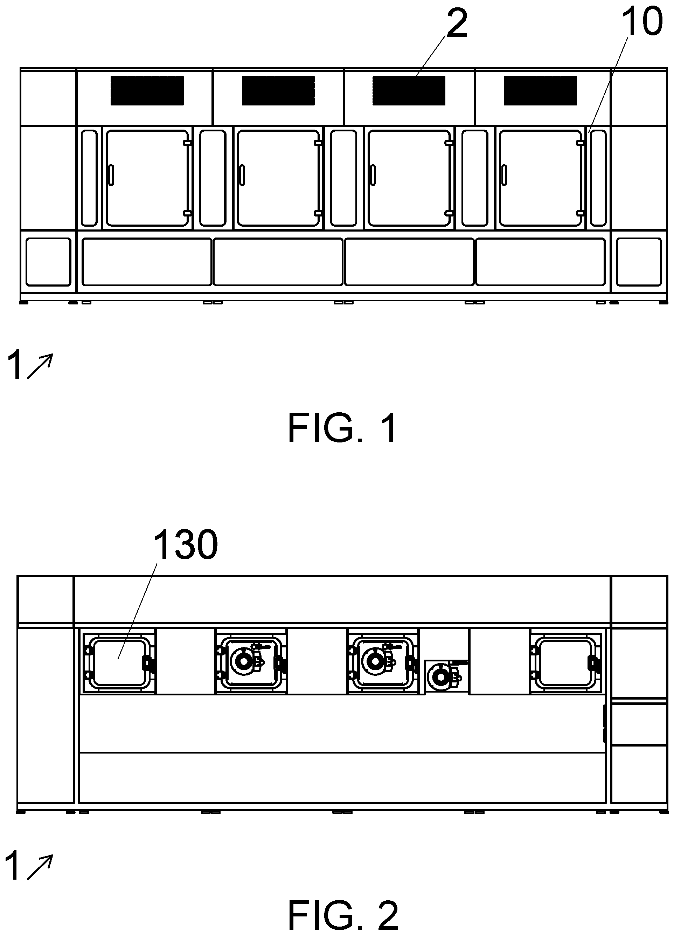

Figure 1 illustrates accesses, for example for maintenance, not strictly necessary. Figure 2 illustrates some openings (130) and the in-view wall provided with openings of the RTP type, an acronym for *Rapid Transfer Port.*

The machine (1) of the invention can comprise further chambers. For example starting from the assembly (0) of figure 10 a single chamber (100) can be realised or a chamber (100) and further chambers, three in the case of figure 14.

The machine (1) realised can comprise three further chambers with an outer conformation that can be the one illustrated in figure 1.

The assembly (0), illustrated in the following, can be made with a "modular" approach guaranteeing one or more stations.

With reference to the machine (1) of figure 3 substantially four areas of fluid velocity or horizontal strips can be observed, starting from above: a first area reaching to the inclined part (133), a second area at the inclined part (133), a third area beneath the inclined part (133) and reaching to the end of the inclination of the second wall (14) and a fourth lower area.

The opening (130) is represented in figure 3, but this might also be present in the other embodiments, as can the fillet (R).

In figures 5 and 6 on the side of the first wall (13) opposite the chamber (100) there is a space in the machine (1) which might be used to arrange a further white chamber or one having a controlled atmosphere, but, also, for the return of the fluid towards the at least an injection mouth (21a, 21b). The teachings of the present invention, with the greater efficiency in cleanliness at the opening (130) also enable reducing the size of the machine (1) on the side of the first wall (13) opposite the chamber (100).

In figure 10, the base (5) comprises, though this is not strictly necessary, a platform which extends from the first part (131). The platform facilitates the transport and management of the assembly (0) before realising the machine (1), while protecting the area with the movement means and, possibly, the functional units (99).

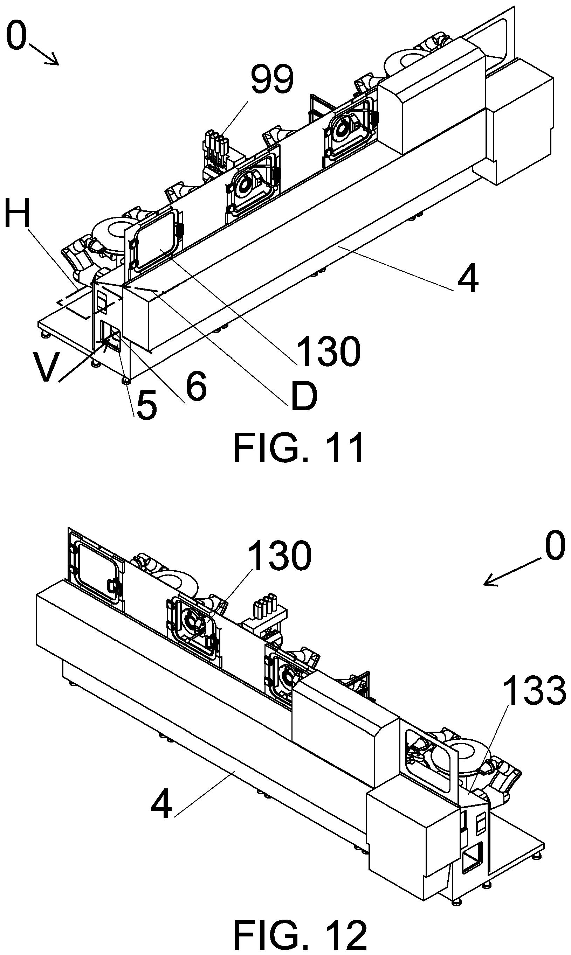

In the same figure 10, as in the following figures 11 and 12, it can be seen how easy it is to access the volume (V) and an electric cable of the means for supplying energy (6) can also been seen.

As illustrated in figures 11 and 12 the assembly (0) can be easily integrated with functional units (99), i.e. modules or devices commonly used in the pharmaceutical or biotechnological industry, ensuring, among other things, a greater facility of access during the installation with respect to installation on a machine (1).

It is understood that the above has been described by way of non-limiting example and that any constructional variants are considered to fall within the protective scope of the present technical solution, as claimed in the following.

## Claims

1. A machine (1) for pharmaceutical or biotechnological processes, comprising:
- a structure (10) which has an upper part (11), a lower part (12) and, between the upper part (11) and lower part (12), walls (13, 14, 15, 16) comprising a first wall (13) and a second wall (14) opposite the first wall (13) and the upper part (11), the lower part (12) and the walls (13, 14, 15, 16) delimiting a chamber (100);
- movement means for moving materials and/or instruments, which are configured to move the materials and/or the instruments in an operating space (100a) internally of the chamber (100);
- ventilation means (2) which comprise at least a fluid injection mouth (21a, 21b) internally of the chamber (100) superiorly of the operating space (100a), at least a fluid extraction mouth (25a, 25b) of fluid from the chamber (100) inferiorly of the operating space (100a) and at least a fluid treatment unit (221) suitable for pharmaceutical or biotechnological processes upstream of the at least an injection mouth (21a, 21b), with the ventilation means (2) moving a fluid from the at least an injection mouth (21a, 21b) to the at least an extraction mouth (25a, 25b);
wherein
the first wall (13) comprises a first part (131), a second part (132) and an inclined part (133) which extends between the first part (131) and the second part (132), inferiorly of the second part (132) and superiorly of the first part (131), and which is contiguous with the first part (131) and with the second part (132) so as to confine the chamber (100);
- the operating space (100a) extends, at least in part, above the inclined part (133) and/or in a volume arranged superiorly of the first part (131) and in front of the inclined part (133);
- the inclined part (133) is inclined with respect to a horizontal plane (H) so that the horizontal section (A) of the chamber (100) crossed by the fluid reduces while descending towards the first part (131),
the machine being **characterised in that**
the structure (10) is configured so that the horizontal section (A) of the chamber (100) is constant, or substantially constant, or diminishes descending from the at least an injection mouth (21a, 21b) to the at least an extraction mouth (25a, 25b);
and **in that** the second part (132) is distanced from the first part (131) according to a horizontal direction (O).

2. The machine of the preceding claim, wherein the first wall (13) and the second wall (14) near one another, inferiorly of the inclined part (133) and superiorly of the at least an extraction mouth (25a, 25b).

3. The machine (1) of any one of the preceding claims, wherein the second part (132) comprises an opening (130) for introducing into or removing materials from the chamber (100)
and wherein the operating space (100a) extends, at least partly, above the inclined part (133).

4. The machine (1) of any one of the preceding claims, wherein an extraction mouth (25a) is made on the second wall (14).

5. The machine (1) of any one of the preceding claims, wherein the at least an extraction mouth (25a, 25b) is made on the first wall (13) and/or according to claim 4 and wherein respectively the second wall (14) and/or the first wall (13) is connected to the lower part (12) with a fillet, wherein the fillet (R) is conformed so as to direct the flow towards the at least an extraction mouth (25a, 25b).

6. The machine (1) of any one of the preceding claims, wherein the upper part (11) houses the at least an injection mouth (21a, 21b) and wherein the ventilation means (2) are configured in such a way that the flow is laminar, or substantially laminar, at least in a first portion (F), and directed perpendicularly to a horizontal plane (H).

7. The machine (1) of the preceding claim, comprising a separator wall (9) of the chamber (100) wherein the separator wall (9) extends from the upper part (11) towards the lower part (12) to a height of the first part (131).

8. The machine (1) of any one of the preceding claims, wherein the at least an injection mouth (21a, 21b) extends, or extends substantially, over all the horizontal section (A) of the chamber (100).

9. The machine (1) of any one of the preceding claims, wherein the first wall (13) and the second wall (14) both have a flat vertical part (134, 141) which descends from the upper part (11) and lies on a respective vertical plane (Z).

10. The machine (1) of any one of the preceding claims, wherein the walls (15, 16) adjacent to the first wall (13) and the second wall (14) are flat and vertical.

11. The machine (1) of any one of the preceding claims, wherein the movement means comprise a part (35) that is coupled to or crosses the first part (131) and/or the inclined part (133).

12. The machine (1) of any one of the preceding claims, comprising means for supplying energy (6) to the movement means and/or means for supplying materials (7) to the movement means arranged at the first part (131) or at the inclined part (133) on the side opposite the chamber (100),
wherein the means for supplying energy (6) and/or the means for supplying materials (7) are located below the inclined part (133).

13. The machine (1) of any one of the preceding claims, comprising at least a functional group (99) which cooperates with the movement means to realise a pharmaceutical or biotechnological process,
wherein the inclined part (133) accommodates or supports at least a functional group (99) of the at least a functional group (99) and/or
wherein the first part (131) supports at least a functional group (99) of the at least a functional group (99).

14. An assembly (0) for realising the machine (1) of any one of the preceding claims, comprising:
- parts of wall for delimiting a chamber (100) comprising a first part (131) and an inclined part (133);
- at least a further wall (4);
- a base (5) which supports the first part (131) and the at least a further wall (4);
- movement means for moving materials and/or instruments, which are configured to move the materials and/or the instruments in an operating space (100a);
- means for supplying energy (6) to the movement means and/or means for supplying materials (7) to the movement means;
wherein:
- the inclined part (133) is contiguous to the first part (131) and rises from the first part (131) in an oblique direction (D) with respect to a horizontal plane (H),
the assembly (O) being **characterised in that** the base (5), the first part (131), the inclined part (133) and the at least a further wall (4) delimit a volume (V) which houses the means for supplying energy (6) and/or the means for supplying materials (7).

15. The assembly (0) of the preceding claim, comprising a separation part (8), wherein:
- the movement means comprise a first device and a second device;
- the separation part (8) is arranged above the inclined part (133) between the first device and the second device and has a first oblique surface (81) and a second oblique surface (82) facing on opposite sides and which broaden towards the inclined part (133) in the conjoining direction (C) of the first device and of the second device.

16. A method for realising the machine (1) according to any one of the preceding claims from 1 to 13, comprising steps of:
- providing an assembly (0) as in claim 14 or 15;
- providing parts comprising an upper part (11), a lower part (12), walls (13, 14, 15, 16) and a second part (132) of wall configured to form a structure (10) together with the parts of wall, with the upper part (11), the lower part (12) and the walls (13, 14, 15, 16) delimiting the chamber (100);
- providing ventilation means (2) which comprise at least a fluid treatment unit (221) suitable for pharmaceutical or biotechnological processes, at least a fluid injection mouth (21a, 21b) and at least a fluid extraction mouth (25a, 25b);
- connecting the parts to the assembly (0) so as to realise a first wall (13) comprising a first part (131), a second part (132) distanced from the first part (131) according to a horizontal direction (O) and an inclined part (133) which is contiguous with the second part (132) and which extends between the first part (131) and the second part (132), inferiorly of the second part (132) and superiorly of the first part (131) and so as to realise a structure (10) having a horizontal section (A) of the chamber (100) which is constant, or substantially constant, or diminishes descending from the installation area of the at least an injection mouth (21a, 21b) to the installation area of the at least an extraction mouth (25a, 25b);
- installing the ventilation means (2) so as to have the at least a fluid injection mouth (21a, 21b) superiorly of the operating space (100a) and the at least a fluid extraction mouth (25a, 25b) from the chamber (100) inferiorly of the operating space (100a) and so as to move a fluid from the at least an injection mouth (21a, 21b) to the at least an extraction mouth (25a, 25b).

17. The method of the preceding claim wherein in the step of providing an assembly (0), an assembly (0) according to claim 15 is provided and wherein in the step of connecting the parts to the assembly (0) a separation part (8) is connected to a wall (13, 14, 15, 16) so that the wall (13, 14, 15, 16) extends between the first oblique surface (81) and a second oblique surface (82).

## Patentansprüche

1. Maschine (1) für pharmazeutische oder biotechnologische Prozesse, umfassend:
- eine Struktur (10), die einen oberen Teil (11), einen unteren Teil (12) und zwischen dem oberen Teil (11) und dem unteren Teil (12) Wände (13, 14, 15, 16) aufweist, die eine erste Wand (13) und eine zweite Wand (14) gegenüber der ersten Wand (13) und dem oberen Teil (11) umfassen, wobei der untere Teil (12) und die Wände (13, 14, 15, 16) eine Kammer (100) begrenzen;
- Bewegungsmittel zum Bewegen von Materialien und/oder Instrumenten, die so konfiguriert sind, dass sie die Materialien und/oder die Instrumente in einem Arbeitsraum (100a) im Inneren der Kammer (100) bewegen;
- Belüftungsmittel (2), die mindestens eine Fluidinjektionsöffnung (21a, 21b) innerhalb der Kammer (100) oberhalb des Arbeitsraums (100a), mindestens eine Fluidentnahmeöffnung (25a, 25b) für Fluid aus der Kammer (100) unterhalb des Arbeitsraums (100a) und mindestens eine Fluidbehandlungseinheit (221), die für pharmazeutische oder biotechnologische Prozesse geeignet ist, stromaufwärts der mindestens einen Injektionsöffnung (21a, 21b), wobei die Belüftungseinrichtung (2) ein Fluid von der mindestens einen Injektionsöffnung (21a, 21b) zu der mindestens einen Extraktionsöffnung (25a, 25b) bewegt;
wobei die erste Wand (13) einen ersten Teil (131), einen zweiten Teil (132) und einen geneigten Teil (133) umfasst, der sich zwischen dem ersten Teil (131) und dem zweiten Teil (132) unterhalb des zweiten Teils (132) und oberhalb des ersten Teils (131) erstreckt und der an den ersten Teil (131) und an den zweiten Teil (132) angrenzt, um die Kammer (100) zu begrenzen;
- der Arbeitsraum (100a) sich zumindest teilweise oberhalb des geneigten Teils (133) und/oder in einem oberhalb des ersten Teils (131) und vor dem geneigten Teil (133) angeordneten Volumen erstreckt;
- der geneigte Teil (133) in Bezug auf eine horizontale Ebene (H) derart geneigt ist, dass der horizontale Querschnitt (A) der Kammer (100), der von dem Fluid durchquert wird, sich beim Abstieg in Richtung des ersten Teils (131) verringert, wobei die Maschine **dadurch gekennzeichnet ist, dass** die Struktur (10) derart konfiguriert ist, dass der horizontale Querschnitt (A) der Kammer (100) konstant oder im Wesentlichen konstant ist oder sich beim Abstieg von der mindestens einen Einspritzmündung (21a, 21b) zu der mindestens einen Entnahmemündung (25a, 25b) verkleinert; und dass der zweite Teil (132) von dem ersten Teil (131) in einer horizontalen Richtung (O) beabstandet ist.

2. Maschine nach dem vorhergehenden Anspruch, bei der die erste Wand (13) und die zweite Wand (14) unterhalb des geneigten Teils (133) und oberhalb der mindestens einen Extraktionsöffnung (25a, 25b) nahe beieinander liegen.

3. Die Maschine (1) nach einem der vorhergehenden Ansprüche, wobei der zweite Teil (132) eine Öffnung (130) zum Einführen von Materialien in die Kammer (100) oder zum Entnehmen von Materialien aus der Kammer (100) aufweist
und wobei sich der Arbeitsraum (100a) zumindest teilweise oberhalb des geneigten Teils (133) erstreckt.

4. Die Maschine (1) nach einem der vorhergehenden Ansprüche, wobei eine Entnahmemündung (25a) an der zweiten Wand (14) ausgebildet ist.

5. Maschine (1) nach einem der vorhergehenden Ansprüche, wobei die mindestens eine Absaugmündung (25a, 25b) an der ersten Wand (13) und/oder nach Anspruch 4 ausgebildet ist und wobei jeweils die zweite Wand (14) und/oder die erste Wand (13) mit dem Unterteil (12) durch eine Ausrundung verbunden ist, wobei die Ausrundung (R) so ausgebildet ist, dass sie die Strömung zu der mindestens einen Absaugmündung (25a, 25b) leitet.

6. Maschine (1) nach einem der vorhergehenden Ansprüche, wobei der obere Teil (11) die mindestens eine Einspritzmündung (21a, 21b) beherbergt und wobei die Belüftungsmittel (2) so gestaltet sind, dass die Strömung zumindest in einem ersten Abschnitt (F) laminar oder im Wesentlichen laminar ist und senkrecht zu einer horizontalen Ebene (H) gerichtet ist.

7. Maschine (1) nach dem vorhergehenden Anspruch, umfassend eine Trennwand (9) der Kammer (100), wobei sich die Trennwand (9) vom oberen Teil (11) zum unteren Teil (12) bis zu einer Höhe des ersten Teils (131) erstreckt.

8. Die Maschine (1) nach einem der vorhergehenden Ansprüche, wobei sich die mindestens eine Einspritzöffnung (21a, 21b) über den gesamten horizontalen Abschnitt (A) der Kammer (100) erstreckt oder im Wesentlichen erstreckt.

9. Maschine (1) nach einem der vorhergehenden Ansprüche, wobei die erste Wand (13) und die zweite Wand (14) jeweils einen flachen vertikalen Teil (134, 141) aufweisen, der von dem oberen Teil (11) abfällt und in einer jeweiligen vertikalen Ebene (Z) liegt.

10. Die Maschine (1) nach einem der vorhergehenden Ansprüche, wobei die an die erste Wand (13) und die zweite Wand (14) angrenzenden Wände (15, 16) flach und vertikal sind.

11. Maschine (1) nach einem der vorhergehenden Ansprüche, wobei die Bewegungseinrichtung ein Teil (35) umfasst, das mit dem ersten Teil (131) und/oder dem geneigten Teil (133) gekoppelt ist oder dieses kreuzt.

12. Maschine (1) nach einem der vorhergehenden Ansprüche, umfassend Mittel zur Zuführung von Energie (6) zu den Bewegungsmitteln und/oder Mittel zur Zuführung von Materialien (7) zu den Bewegungsmitteln, die an dem ersten Teil (131) oder an dem schrägen Teil (133) auf der der Kammer (100) gegenüberliegenden Seite angeordnet sind,
wobei die Mittel zur Energiezufuhr (6) und/oder die Mittel zur Materialzufuhr (7) unterhalb des schrägen Teils (133) angeordnet sind.

13. Maschine (1) nach einem der vorhergehenden Ansprüche, umfassend mindestens eine Funktionsgruppe (99), die mit den Bewegungsmitteln zusammenwirkt, um einen pharmazeutischen oder biotechnologischen Prozess zu realisieren,
wobei der geneigte Teil (133) mindestens eine Funktionsgruppe (99) der mindestens einen Funktionsgruppe (99) aufnimmt oder trägt und/oder
wobei der erste Teil (131) mindestens eine funktionelle Gruppe (99) der mindestens einen funktionellen Gruppe (99) trägt.

14. Baugruppe (0) zur Realisierung der Maschine (1) nach einem der vorhergehenden Ansprüche, umfassend:
- Wandteile zur Begrenzung einer Kammer (100) mit einem ersten Teil (131) und einem geneigten Teil (133);
- mindestens eine weitere Wand (4);
- eine Basis (5), die den ersten Teil (131) und die mindestens eine weitere Wand (4) trägt;
- Bewegungsmittel zum Bewegen von Materialien und/oder Instrumenten, die zum Bewegen der Materialien und/oder der Instrumente in einem Arbeitsraum (100a) konfiguriert sind;
- Mittel zum Zuführen von Energie (6) zu den Bewegungsmitteln und/oder Mittel zum Zuführen von Materialien (7) zu den Bewegungsmitteln;
wobei:
- der geneigte Teil (133) an den ersten Teil (131) angrenzt und von dem ersten Teil (131) in einer schrägen Richtung (D) in Bezug auf eine horizontale Ebene (H) ansteigt, wobei die Anordnung (O) **dadurch gekennzeichnet ist, dass** die Basis (5), der erste Teil (131), der geneigte Teil (133) und die mindestens eine weitere Wand (4) ein Volumen (V) begrenzen, das die Mittel zur Energiezufuhr (6) und/oder die Mittel zur Materialzufuhr (7) aufnimmt.

15. Baugruppe (0) nach dem vorhergehenden Anspruch, umfassend ein Trennteil (8), wobei:
- die Bewegungsmittel eine erste Vorrichtung und eine zweite Vorrichtung umfassen;
- das Trennteil (8) oberhalb des schrägen Teils (133) zwischen der ersten Vorrichtung und der zweiten Vorrichtung angeordnet ist und eine erste Schrägfläche (81) und eine zweite Schrägfläche (82) aufweist, die auf gegenüberliegenden Seiten liegen und sich in Richtung des schrägen Teils (133) in der Verbindungsrichtung (C) der ersten Vorrichtung und der zweiten Vorrichtung verbreitern.

16. Verfahren zur Realisierung der Maschine (1) nach einem der vorhergehenden Ansprüche 1 bis 13, umfassend die Schritte:
- Bereitstellen einer Baugruppe (0) nach Anspruch 14 oder 15;
- Bereitstellen von Teilen, die ein Oberteil (11), ein Unterteil (12), Wände (13, 14, 15, 16) und ein zweites Wandteil (132) umfassen, die so gestaltet sind, dass sie zusammen mit den Wandteilen eine Struktur (10) bilden, wobei das Oberteil (11), das Unterteil (12) und die Wände (13, 14, 15, 16) die Kammer (100) begrenzen;
- Vorsehen von Belüftungsmitteln (2), die mindestens eine für pharmazeutische oder biotechnologische Prozesse geeignete Fluidbehandlungseinheit (221), mindestens eine Fluidinjektionsöffnung (21a, 21b) und mindestens eine Fluidentnahmeöffnung (25a, 25b) umfassen;
- Verbinden der Teile mit der Einheit (0), um eine erste Wand (13) zu realisieren, die einen ersten Teil (131), einen zweiten Teil (132), der von dem ersten Teil (131) gemäß einer horizontalen Richtung (O) beabstandet ist, und einen geneigten Teil (133) umfasst, der an den zweiten Teil (132) angrenzt und der sich zwischen dem ersten Teil (131) und dem zweiten Teil (132) erstreckt, unterhalb des zweiten Teils (132) und oberhalb des ersten Teils (131) erstreckt, um eine Struktur (10) zu realisieren, die einen horizontalen Querschnitt (A) der Kammer (100) aufweist, der konstant oder im Wesentlichen konstant ist oder vom Installationsbereich der mindestens einen Einspritzmündung (21a, 21b) zum Installationsbereich der mindestens einen Entnahmemündung (25a, 25b) hin abnimmt;
- Installieren der Belüftungseinrichtung (2), so dass die mindestens eine Fluideinspritzmündung (21a, 21b) oberhalb des Arbeitsraums (100a) und die mindestens eine Fluidabsaugmündung (25a, 25b) von der Kammer (100) unterhalb des Arbeitsraums (100a) liegt, und so, dass ein Fluid von der mindestens einen Einspritzmündung (21a, 21b) zu der mindestens einen Absaugmündung (25a, 25b) bewegt wird.

17. Verfahren nach dem vorhergehenden Anspruch, wobei im Schritt des Bereitstellens einer Baugruppe (0) eine Baugruppe (0) nach Anspruch 15 bereitgestellt wird und wobei im Schritt des Verbindens der Teile mit der Baugruppe (0) ein Trennteil (8) mit einer Wand (13, 14, 15, 16) verbunden wird, so dass sich die Wand (13, 14, 15, 16) zwischen der ersten Schrägfläche (81) und einer zweiten Schrägfläche (82) erstreckt.

## Revendications

1. Une machine (1) pour des processus pharmaceutiques ou biotechnologiques, comprenant :
- une structure (10) qui présente une partie supérieure (11), une partie inférieure (12) et, entre la partie supérieure (11) et la partie inférieure (12), des parois (13, 14, 15, 16) comprenant une première paroi (13) et une seconde paroi (14) opposée à la première paroi (13) et à la partie supérieure (11), la partie inférieure (12) et les parois (13, 14, 15, 16) délimitant une chambre (100);
- des moyens de déplacement de matériaux et/ou d'instruments, configurés pour déplacer les matériaux et/ou les instruments dans un espace de travail (100a) à l'intérieur de la chambre (100) ;
- des moyens de ventilation (2) comprenant au moins une bouche d'injection de fluide (21a, 21b) à l'intérieur de la chambre (100), au-dessus de l'espace de travail (100a), au moins une bouche d'extraction de fluide (25a, 25b) de fluide provenant de la chambre (100) au-dessous de l'espace de fonctionnement (100a) et au moins une unité de traitement de fluide (221) adaptée aux processus pharmaceutiques ou biotechnologiques en amont de la bouche d'injection (21a, 21b), les moyens de ventilation (2) déplaçant un fluide de la bouche d'injection (21a, 21b) à la bouche d'extraction (25a, 25b) ;
dans laquelle la première paroi (13) comprend une première partie (131), une deuxième partie (132) et une partie inclinée (133) qui s'étend entre la première partie (131) et la deuxième partie (132), en dessous de la deuxième partie (132) et au-dessus de la première partie (131), et qui est contiguë à la première partie (131) et à la deuxième partie (132) de manière à confiner la chambre (100) ;
- l'espace de travail (100a) s'étend, au moins en partie, au-dessus de la partie inclinée (133) et/ou dans un volume disposé au-dessus de la première partie (131) et devant la partie inclinée (133) ;
- la partie inclinée (133) est inclinée par rapport à un plan horizontal (H) de sorte que la section horizontale (A) de la chambre (100) traversée par le fluide diminue en descendant vers la première partie (131), la machine étant **caractérisée en ce que** la structure (10) est configurée pour que la section horizontale (A) de la chambre (100) soit constante, ou sensiblement constante, ou diminue en descendant depuis au moins une bouche d'injection (21a, 21b) jusqu'à au moins une bouche d'extraction (25a, 25b) ; et **en ce que** la deuxième partie (132) est distante de la première partie (131) selon une direction horizontale (O).

2. La machine de la revendication précédente, dans laquelle la première paroi (13) et la deuxième paroi (14) sont proches l'une de l'autre, en dessous de la partie inclinée (133) et au-dessus d'au moins une bouche d'extraction (25a, 25b).

3. La machine (1) de l'une quelconque des revendications précédentes, dans laquelle la deuxième partie (132) comprend une ouverture (130) pour introduire ou retirer des matériaux de la chambre (100)
et dans laquelle l'espace de travail (100a) s'étend, au moins en partie, au-dessus de la partie inclinée (133).

4. La machine (1) de l'une quelconque des revendications précédentes, dans laquelle une bouche d'extraction (25a) est pratiquée sur la deuxième paroi (14).

5. La machine (1) de l'une quelconque des revendications précédentes, dans laquelle au moins une bouche d'extraction (25a, 25b) est réalisée sur la première paroi (13) et/ou selon la revendication 4 et dans laquelle respectivement la deuxième paroi (14) et/ou la première paroi (13) est reliée à la partie inférieure (12) par un congé, le congé (R) étant conformé de manière à diriger l'écoulement vers au moins une bouche d'extraction (25a, 25b).

6. La machine (1) de l'une quelconque des revendications précédentes, dans laquelle la partie supérieure (11) abrite au moins une bouche d'injection (21a, 21b) et dans laquelle les moyens de ventilation (2) sont configurés de manière à ce que le flux soit laminaire, ou sensiblement laminaire, au moins dans une première portion (F), et dirigé perpendiculairement à un plan horizontal (H).

7. La machine (1) de la revendication précédente, comprenant une paroi séparatrice (9) de la chambre (100) dans laquelle la paroi séparatrice (9) s'étend de la partie supérieure (11) vers la partie inférieure (12) à une hauteur de la première partie (131).

8. La machine (1) de l'une quelconque des revendications précédentes, dans laquelle au moins une bouche d'injection (21a, 21b) s'étend, ou s'étend sensiblement, sur toute la section horizontale (A) de la chambre (100).

9. La machine (1) de l'une quelconque des revendications précédentes, dans laquelle la première paroi (13) et la deuxième paroi (14) ont toutes deux une partie verticale plate (134, 141) qui descend de la partie supérieure (11) et se trouve sur un plan vertical respectif (Z).

10. La machine (1) de l'une quelconque des revendications précédentes, dans laquelle les parois (15, 16) adjacentes à la première paroi (13) et à la seconde paroi (14) sont plates et verticales.

11. La machine (1) de l'une quelconque des revendications précédentes, dans laquelle les moyens de déplacement comprennent une partie (35) qui est couplée à ou traverse la première partie (131) et/ou la partie inclinée (133).

12. La machine (1) de l'une quelconque des revendications précédentes, comprenant des moyens pour fournir de l'énergie (6) aux moyens de mouvement et/ou des moyens pour fournir des matériaux (7) aux moyens de mouvement disposés sur la première partie (131) ou sur la partie inclinée (133) du côté opposé à la chambre (100),
dans lequel les moyens d'alimentation en énergie (6) et/ou les moyens d'alimentation en matériaux (7) sont situés en dessous de la partie inclinée (133).

13. La machine (1) de l'une quelconque des revendications précédentes, comprenant au moins un groupe fonctionnel (99) qui coopère avec les moyens de mouvement pour réaliser un processus pharmaceutique ou biotechnologique,
dans laquelle la partie inclinée (133) accueille ou supporte au moins un groupe fonctionnel (99) de l'au moins un groupe fonctionnel (99) et/ou
dans laquelle la première partie (131) supporte au moins un groupe fonctionnel (99) du groupe fonctionnel (99).

14. Ensemble (0) pour la réalisation de la machine (1) de l'une quelconque des revendications précédentes, comprenant :
- des parties de paroi pour délimiter une chambre (100) comprenant une première partie (131) et une partie inclinée (133) ;
- au moins une autre paroi (4) ;
- une base (5) qui supporte la première partie (131) et au moins une autre paroi (4) ;
- des moyens de mouvement pour déplacer des matériaux et/ou des instruments, qui sont configurés pour déplacer les matériaux et/ou les instruments dans un espace de travail (100a) ;
- des moyens pour fournir de l'énergie (6) aux moyens de déplacement et/ou des moyens pour fournir des matériaux (7) aux moyens de déplacement ;
dans lequel :
- la partie inclinée (133) est contiguë à la première partie (131) et s'élève à partir de la première partie (131) dans une direction oblique (D) par rapport à un plan horizontal (H), l'ensemble (O) étant **caractérisé en ce que** la base (5), la première partie (131), la partie inclinée (133) et au moins une autre paroi (4) délimitent un volume (V) qui abrite les moyens d'alimentation en énergie (6) et/ou les moyens d'alimentation en matériaux (7).

15. L'ensemble (0) de la revendication précédente, comprenant une partie de séparation (8), dans lequel :
- les moyens de déplacement comprennent un premier dispositif et un deuxième dispositif ;
- la partie de séparation (8) est disposée au-dessus de la partie inclinée (133) entre le premier dispositif et le deuxième dispositif et présente une première surface oblique (81) et une deuxième surface oblique (82) tournées vers des côtés opposés et qui s'élargissent vers la partie inclinée (133) dans la direction de jonction (C) du premier dispositif et du deuxième dispositif.

16. Procédé de réalisation de la machine (1) selon l'une quelconque des revendications précédentes de 1 à 13, comprenant les étapes de :
- fournir un ensemble (0) selon la revendication 14 ou 15 ;
- fournir des pièces comprenant une partie supérieure (11), une partie inférieure (12), des parois (13, 14, 15, 16) et une deuxième partie (132) de paroi configurée pour former une structure (10) avec les parties de paroi, la partie supérieure (11), la partie inférieure (12) et les parois (13, 14, 15, 16) délimitant la chambre (100) ;
- prévoir des moyens de ventilation (2) qui comprennent au moins une unité de traitement des fluides (221) adaptée aux procédés pharmaceutiques ou biotechnologiques, au moins une bouche d'injection des fluides (21a, 21b) et au moins une bouche d'extraction des fluides (25a, 25b) ;
- relier les pièces à l'ensemble (0) de manière à réaliser une première paroi (13) comprenant une première partie (131), une deuxième partie (132) distante de la première partie (131) selon une direction horizontale (O) et une partie inclinée (133) qui est contiguë à la deuxième partie (132) et qui s'étend entre la première partie (131) et la deuxième partie (132), inférieurement à la deuxième partie (132) et supérieurement à la première partie (131) et de manière à réaliser une structure (10) ayant une section horizontale (A) de la chambre (100) qui est constante, ou sensiblement constante, ou décroissante depuis la zone d'installation d'au moins une bouche d'injection (21a, 21b) jusqu'à la zone d'installation d'au moins une bouche d'extraction (25a, 25b) ;
- installer le moyen de ventilation (2) de manière à avoir au moins une bouche d'injection de fluide (21a, 21b) au-dessus de l'espace de travail (100a) et au moins une bouche d'extraction de fluide (25a, 25b) de la chambre (100) au-dessous de l'espace de travail (100a) et de manière à déplacer un fluide de la au moins une bouche d'injection (21a, 21b) à la au moins une bouche d'extraction (25a, 25b).

17. Procédé de la revendication précédente dans lequel, lors de l'étape de fourniture d'un ensemble (0), un ensemble (0) selon la revendication 15 est fourni et dans lequel, lors de l'étape de connexion des pièces à l'ensemble (0), une pièce de séparation (8) est connectée à une paroi (13, 14, 15, 16) de sorte que la paroi (13, 14, 15, 16) s'étende entre la première surface oblique (81) et une deuxième surface oblique (82).
